# EUROPEAN PATENT APPLICATION

(11) **EP 2 108 365 A1**
(43) Date of publication of application: **14.10.2009**
(21) Application number: 08154239.1
(22) Date of filing: 09.04.2008
(51) Int. Cl.: A61K 9/16, A61K 9/20, A61K 31/4178

(54) **Single dosage pharmaceutical formulation comprising eprosartan mesylate**

(71) Applicant: LEK Pharmaceuticals d.d., 1526 Ljubljana (SI)
(72) Inventor: The designation of the inventor has not yet been filed
(74) Representative: Kröger, Bernd

(57) **Abstract**

A dry formulation or granulation of eprosartan mesylate is described which comprises eprosartan mesylate in particulate form with a particle size, wherein at least 65 v/v % eprosartan mesylate particles fall in a particle size range of from 2 to 27 µm. In another aspect, a dry formulation or granulation of eprosartan mesylate comprises eprosartan mesylate combined with an excipient which at least comprises a PEG having molecular weight in the range of 400 to 20000 and mannitol. Further described is a single dosage pharmaceutical formulation such as tablet obtained from such a dry formulation or granulation of eprosartan mesylate by direct compression or dry granulation. A dry formulation or granulation of eprosartan mesylate, or a process for the preparation thereof is also described, which comprising eprosartan mesylate in particulate form mixed with one or more excipients or additives in a way that a limited water activity is obtained. The dry formulation or granulation of eprosartan mesylate can be directly compressed or processed by dry granulation, while maintaining the eprosartan mesylate in only one stable form. Suitable prophylactic and/or therapeutic uses are also described.

## Description

### Field of the Invention

The present invention relates to a new dry formulation or granulation of eprosartan mesylate, and to a single dosage pharmaceutical formulation obtained therefrom. The present invention relates to a new process for the preparation of dry formulation or granulation of eprosartan mesylate. The single dosage pharmaceutical formulation is particularly useful as a medicament, especially for prophylaxis and/or treatment of hypertension, congestive heart failure and renal failure.

### Description of Background Art

Eprosartan mesylate, chemically (E)-α-[2-n-butyl-1-[(4-carboxyphenyl)methyl]-1H-imidazol-5-yl]methylene-2-thiophenepropionic acid monomethanesulfonate, is a angiotensin II receptor (AT₁) antagonist approved for the treatment of essential hypertension. The drug is a well tolerated and effective antihypertensive agent with benefit in the secondary prevention of cerebrovascular events, independent of blood pressure (BP)-lowering effects. Eprosartan mesylate has a low potential for serious adverse events and has not been associated with clinically significant drug interactions, establishing it as a promising agent for combination antihypertensive strategies.

Due to low solubility (typically less than 0.1 mg/ml at pH 2) and low permeability across the intestinal epithelium the drug exhibits very low bioavailability (13%). Therefore, high dose of eprosartan mesylate needs to be incorporated in the oral pharmaceutical dosage form in order to achieve the desired biological effect (i.e. significant reduction of blood pressure). The recommended daily starting and usual maintainance dose of eprosartan mesylate when used as monotherapy is 735.8 mg, equivalent to 600 mg eprosartan, available as the commercial Teveten^{™} 600 mg tablets. Therefore, the amount of excipients, required to achieve satisfactory technological properties (i.e. flowability, compressibility) should be the least possible to keep the weight of the tablet as low as possible (desirably less than about 1000 mg). Larger tablets are less preferred in terms of patient compliance.

US 6,262,102 B1 relates to a monohydrate form, and US 2001/0031877 A1 relates to the dihydrate form of eprosartan and processes for their production, respectively. According to US 6,262,102 B1, the monohydrate form is produced during vacuum drying of the dihydrated form, or when the anhydrate is granulated with water, stored and vacuum dried. According to US 2001/0031877 A1, the dihydrated form is prepared *in situ* during the wet granulation process of the anhydrous form of the compound. The pharmaceutical formulations disclosed in the above patents are all produced by wet granulation, starting from anhydrous eprosartan mesylate, allowing the various hydrate forms to arise in situ, rendering the process unpredictable and leaving the formulation with the presence of various forms of eprosartan mesylate, namely anhydrous, monohydrate and dihydrate. In US 2002/0098241 A1, it is described that the free base form - distinct from the mesylate salt form - of anhydrous eprosartan does not form a hydrate during wet granulation, and therefore free base anhydrous form of eprosartan is proposed for a wet or dry granulation with high drug dose.

### Summary of the invention

The object of the present invention was to provide an improved eprosartan mesylate formulation or granulation with a physically stable eprosartan mesylate, and to make eprosartan mesylate dosage pharmaceutical formulations, in particular tablet dosage forms, as well as a production process thereof more robust, economical and acceptable.

In one aspect the present invention provides a dry formulation or granulation of eprosartan mesylate, comprising eprosartan mesylate in particulate form, wherein at least 65 v/v % eprosartan mesylate particles fall in a particle size range of from 2 to 27 µm.

In a further aspect the present invention provides a dry formulation or granulation of eprosartan mesylate, comprising eprosartan mesylate combined with an excipient, wherein the excipient at least comprises a PEG having molecular weight in the range of 400 to 20000 and mannitol.

In another aspect, the present invention further provides a pharmaceutical formulation, in particular a single dosage form such as one or more tablets, obtained from the aforementioned dry formulation or dry granulation of eprosartan mesylate by direct compression or dry granulation.

The present invention further provides a pharmaceutical formulation comprising eprosartan mesylate, which has a dissolution profile by releasing eprosartan at a rate of at least 50 % within 30 minutes, relative to the original amount of eprosartan in the formulation, tested using USP apparatus 2, placing the formulations in 1000 ml 0.1 M hydrochloric acid at 37 ± 0.5°C with paddle speed of 50 rpm.

The present invention still further provides a set of pharmaceutical formulation samples each comprising eprosartan mesylate as an active ingredient, wherein the eprosartan mesylate shows a dissolution profile with a variability of dissolution from the different pharmaceutical formulation samples of the set of below 30 %, preferably below 20% and more preferably below 10% relative standard deviation at all time during dissolution, measured using USP apparatus 2, placing the formulations in 1000 ml 0.1 M hydrochloric acid at 37 ± 0.5°C with paddle speed of 50 rpm.

In another aspect, the present invention further provides a dry formulation or granulation of eprosartan mesylate, comprising eprosartan mesylate in particulate form mixed with excipients or additives, wherein the dry formulation or granulation of eprosartan mesylate has a water activity of less than 0.62.

When the aforementioned step of mixing further comprises a step of direct compression, or a step of dry granulation of the mixed components, a pharmaceutical formulation of eprosartan mesylate can be prepared.

In a still further aspect, the present invention provides a process for the preparation of a pharmaceutical formulation of eprosartan mesylate, wherein eprosartan mesylate in only one primary form of being either anhydrous or dihydrate is provided, and said primary form is subjected to a direct compression or a dry granulation process while maintaining said only one primary form.

Furthermore, the present invention provides a package comprising at least one single dosage form comprising eprosartan mesylate in only one form of either anhydrous or dihydrate form, which single dosage form was obtained through dry granulation or direct compression; wherein said at least one single dosage form is packed in a package sealed against vapor and moisture permeation.

The pharmaceutical formulation and the blister pack provided or prepared according to the present invention is particularly suitable for prophylactic and/or therapeutic treatment of hypertension, congestive heart failure and renal failure.

In the above-mentioned aspects, eprosartan mesylate is preferably in anhydrous form, more preferably in the essential and especially in the total absence of monohydrate and dihydrate forms of eprosartan mesylate. Absence especially means that neither the monohydrate form nor the dihydrate form of eprosartan mesylate is detectable by X-ray observation.

### Description of the invention, its advantages and preferred embodiments:

The aspects, advantageous features and preferred embodiments of the present invention summarized in the following items, respectively alone or in combination, further contribute to solving the object of the invention:
(1) A dry formulation or granulation of eprosartan mesylate, comprising eprosartan mesylate in particulate form, wherein at least 65 v/v % eprosartan mesylate particles fall in a particle size range of from 2 to 27 µm.
(2) The dry formulation or granulation of eprosartan mesylate according to (1), wherein at least 75 v/v %, preferably at least 85 v/v % eprosartan mesylate particles fall in a particle size range of from 2 to 27 µm.
(3) The dry formulation or granulation of eprosartan mesylate according to (1) or (2), wherein the excipient comprises an additive selected from the group consisting of polyethyleneglycol (PEG; Macrogol), preferably PEGs having molecular weights in the range of 400 to 20000; polyvinyl pyrrolidone (PVP) or its cross-linked form crospovidone; silicon dioxide, preferably fumed silica or colloidal silicon dioxide; sugar esters and polyhydroxy-sugars, preferably mannitol; lactose, isomalt, lactitol and dextrose; buffer salts, preferably anhydrous mono-, di- or tri-basic phosphate, more preferably calcium phosphate, calcium sulfate and calcium silicate; hydrophilic, anionic, cationic, zwitterionic and non-ionic surfactants and lipohilic additives such as mono-, di- or triglycerides, polyethoxylated fatty acids, fatty acid esters and oils, preferably sodium oleate, sodium lauryl sulfate and magnesium stearate and polyglycolized glycerides; talc; polysaccharides, preferably and starch and celluloses, more preferably hydroxyethylcellulose (HEC), hydroxypropylcellulose (HPC), hydroxypropyl methylcellulose (HPMC), HPMC phthalate, cyclodextrins and carbomers, in particular hydroxypropyl methylcellulose, microcrystalline cellulose (MCC), powdered cellulose; polypeptides such as gelatine; and mixtures thereof.
(4) The dry formulation or granulation of eprosartan mesylate according to any one of (1) to (3), wherein the excipient comprises an additive selected from the group consisting of PEGs having molecular weight in the range of 400 to 20000; MCC having a particle size of about 50 to about 100 µm and having a water content of ≤ 1.5 %; fumed silica having a specific surface area of at least about 50 m²/g, and mixtures thereof.
(5) The dry formulation or granulation of eprosartan mesylate according to any one of (1) to (4), wherein the excipient comprises an additive selected from the group consisting of lactose, isomalt and mannitol, and mixtures thereof.
(6) A dry formulation or granulation of eprosartan mesylate, comprising eprosartan mesylate combined with an excipient, wherein the excipient at least comprises a PEG having molecular weight in the range of 400 to 20000 and mannitol.
(7) The dry formulation or granulation of eprosartan mesylate according to (6), wherein the excipient further comprises lactose, isomalt, or mixtures thereof.
(8) The dry formulation or granulation of eprosartan mesylate according to any one of (1) to (7), wherein none of excipients or additives added to the formulation or granulation are combined with agglomeration liquid and all the excipients or additives are added in the form of powders optionally with the exception of appropriate excipients or additives that do not exist in powdery form.
(9) The dry formulation or granulation of eprosartan mesylate according to any one of (1) to (8), wherein the respective amounts of each excipient or additive does not exceed 10 wt.-%.
(10) The dry formulation or granulation of eprosartan mesylate according to any one of (1) to (9), wherein said eprosartan mesylate is in dihydrate form and preferably in anhydrous form only, without presence of other forms of eprosartan mesylate.
(11) The dry formulation or granulation of eprosartan mesylate according to any one of (1) to (10) , which has a total water activity of less than 0.62, preferably less than 0.60 and more preferably less than 0.50, respectively determined at room temperature.
(12) The dry formulation or granulation of eprosartan mesylate according to any one of (1) to (11), which has a water content of ≤5 wt.-%, preferably ≤2 wt.-% and particularly ≤0.5 wt.-% of the whole dry formulation or granulation.
(13) The dry formulation or granulation of eprosartan mesylate according to any one of (1) to (12), which is defined by a granulation having a particle size distribution in which 40 wt.-% or less of the particles are smaller than 45 µm, preferably 35 wt.-% or less of the particles and more preferably 20 wt.-% or less of the particles are smaller than 45 µm.
(14) The dry formulation or granulation of eprosartan mesylate according to any one of (1) to (13), capable of being directly compressed into eprosartan mesylate tablets.
(15) The dry formulation or granulation of eprosartan mesylate according to any one of (1) to (14), which has a compressibility of at least 40 %, preferably at least 35%, and in particular at least 30 %.
(16) A pharmaceutical formulation, obtained from a dry formulation or granulation of eprosartan mesylate according to any one of (1) to (15) by direct compression or dry granulation.
(17) The pharmaceutical formulation according to (16), which is formulated in a single dosage form, preferably a tablet.
(18) The pharmaceutical formulation according to (16) or (17), wherein the eprosartan mesylate is physically stable such that prevention of an interconversion from a first form of being either anhydrous or di-hydrated to another or mixed form of eprosartan mesylate is essentially maintained over a period of at least 3 months, preferably at least 6 months.
(19) The pharmaceutical formulation according to any one of (16) to (18), comprising eprosartan mesylate at an amount of at least 50 %, preferably at least 65 %, particularly at least 70 % w/w relative to the total amount of the single dosage pharmaceutical formulation.
(20) The pharmaceutical formulation according to any one of (16) to (19), which has a hardness of 80 to 280 N, particularly 100 to 250 N, more particularly 150 to 200 N.
(21) A pharmaceutical formulation comprising eprosartan mesylate, which has a dissolution profile by releasing eprosartan at a rate of at least 50 % within 30 minutes, preferably releasing eprosartan at a rate of at least 60 % within 60 minutes and at least 70 % within 90 minutes, respectively relative to the original amount of eprosartan in the formulation, tested using USP apparatus 2, placing the formulations in 1000 ml 0.1 M hydrochloric acid at 37 ± 0.5°C with paddle speed of 50 rpm., relative to the original amount of eprosartan in the formulation
(22) A set of pharmaceutical formulation samples, wherein each comprising eprosartan mesylate as an active ingredient, wherein the eprosartan mesylate shows a dissolution profile with a variability of dissolution from different pharmaceutical formulation samples of the set of below 30 %, preferably below 20% and more preferably below 10% relative standard deviation at all time during dissolution, measured using USP apparatus 2, placing the formulations in 1000 ml 0.1 M hydrochloric acid at 37 ± 0.5°C with paddle speed of 50 rpm.
(23) The pharmaceutical formulation according to (21) or the set of pharmaceutical formulation samples according to (22), each comprising eprosartan mesylate in only either anhydrous or dihydrate form as an active ingredient.
(24) The pharmaceutical formulation according to (21) or the set of pharmaceutical formulation samples according to (22), further defined as set forth in any one of (16) to (20).
(25) A dry formulation or granulation of eprosartan mesylate, or a process for the preparation thereof, comprising eprosartan mesylate in particulate form mixed with excipients or additives, wherein the prepared whole dry formulation or granulation of eprosartan mesylate has a water activity of less than 0.62, preferably less than 0.60 and more preferably less than 0.50, respectively determined at room temperature.
(26) The dry formulation or granulation, or the process according to (25), wherein each excipient or additive used for the preparation has a limited water activity of less than 0.62, preferably less than 0.60 and more preferably less than 0.50, respectively determined at room temperature.
(27) The dry formulation or granulation, or the process according to (25) or (26), wherein the excipients or additives used in the mixing step are as defined in any one of (3) to (9).
(28) The dry formulation or granulation, or the process according to any one of (25) to (27), wherein the particulate eprosartan mesylate used in the mixing step is as defined in any one of (1), (2) and (10).
(29) The dry formulation or granulation, or the process according to any one of (25) to (28), wherein the mixing step is performed under essentially water-free conditions defined by a water content of ≤5 wt.-%, preferably ≤2 wt.-% and particularly ≤0.5 wt.-% of the dry formulation or granulation.
(30) A pharmaceutical formulation of eprosartan mesylate, or a process for the preparation thereof, comprising a dry formulation or granulation of eprosartan mesylate or a process for the preparation thereof as defined in any one of (25) to (29), and further processed by direct compression of the mixed components.
(31) A pharmaceutical formulation of eprosartan mesylate, or a process for the preparation thereof, comprising a dry formulation or granulation of eprosartan mesylate or a process for the preparation thereof as defined in any one of (25) to (29), and further processed by dry granulation.
(32) The pharmaceutical formulation or process according to any one of (30) to (31), further processed by briquetting or slugging, then optionally sieving or milling, and subsequently tabletting, to thereby obtain single dosage forms, preferably tablets.
(33) The pharmaceutical formulation or process according to (32), wherein the step of briquetting or slugging, then optionally sieving or milling, is performed to control produced particle size showing a particle size distribution defined by less than 40 wt-%, preferably less than wt.-35 % and more preferably less than 20 wt.-% of the particles being smaller than 45 µm.
(34) A pharmaceutical formulation of eprosartan mesylate, or a process for the preparation thereof, obtained by providing eprosartan mesylate in only one primary form of being either anhydrous or dihydrate, and subjecting said primary form to a direct compression or dry granulation process while maintaining said only one primary form.
(35) The pharmaceutical formulation or process according to (34), characterized by providing and subsequently maintaining only anhydrous eprosartan mesylate.
(36) The pharmaceutical formulation or process according to (34) or (35), wherein the produced primary form of eprosartan mesylate is not interconverted to another form of eprosartan mesylate over a period of at least 3 months, preferably at least 6 months.
(37) The pharmaceutical formulation or process according to one of (34) to (36), further defined according to any one of (25) to (33).
(38) A package comprising: at least one single dosage form comprising eprosartan mesylate in only one form of either anhydrous or dihydrate form, which single dosage form was obtained through dry granulation or direct compression; wherein said at least one single dosage form is packed in a package sealed against vapor and moisture permeation, and preferably further protected against light exposure.
(39) The package according to (38), which is designed as a blister pack.
(40) The package according to (39), which is designed as bottles made mainly or completely of HDPE (high density polyethylene).
(41) The package according to one of (38) to (40), which is sealed against vapor permeation by forming a foil/foil blister, preferably an aluminum/aluminum blister, or by forming a pack comprising a blister base part and a cover film consisting of aluminum or an aluminum/plastics material composite, and a lower sealing tray, which is formed from an aluminum/plastics material laminate, being sealed against the rear of the blister base part.
(42) Use of the items according to any one of (16) to (41) for prophylactic and/or therapeutic treatment of hypertension, congestive heart failure and/or renal failure.

It was surprisingly found that by using eprosartan mesylate in particulate form having a certain limited particle size range, a dry formulation or granulation comprising this eprosartan mesylate form is improved in free-flowing and cohesive powder characteristics and displays cohesive briquetting and dry granulation behaviour, which effects are significant for efficient and robust dry granulation tableting or direct compression. When too much eprosartan mesylate particles are present whose particle size is lower than about 2 µm, picking and sticking tend to be caused in the later processing, and when too much eprosartan mesylate particles are present whose particle size is larger than about 27 µm, the compressibility tends to become poor. Therefore, it is observed that at least 65 v/v % eprosartan mesylate particles of the particle size distribution fall in a particle size range of from 2 to 27 µm. Moreover, observing the specific particle size range of the starting eprosartan mesylate in the particulate form, especially when in the anhydrous form, contributes to a remarkably enhanced dissolution profile and to a high dissolution reproducibility in terms of low variability of dissolution rate. When processing eprosartane mesylate, its particle size remains substantially unchanged, typically it remains totally unchanged. In view of improved overall characteristics, it is preferred that at least 75 v/v %, more preferably at least 85 v/v % of the eprosartan mesylate particles processed according to the present invention fall in a particle size range of from 2 to 27 µm.

It was further found that using eprosartan mesylate, particularly when used in anhydrous form, in combination with particular excipients, which at least comprise PEG in the range of 400 to 20000, in particular macrogol 4000 (PEG-4000), together with mannitol, a surprisingly remarkable positive influence on dissolution profiles in *in vitro* studies is obtained, and further physical properties of a powder mixture for briquetting and granulate for tableting are significantly enhanced with both of these excipients, subsequently providing good compressibility and less weight and hardness variability of resulting tablets.

Furthermore, it has been surprisingly found that by processing eprosartan mesylate, particularly when used in anhydrous form, with excipients or additives respectively having such a water activity, that their cumulative contribution to the final water activity of a final dosage form is less than 0.62, a dry formulation or granulation preparation of anhydrous eprosartan mesylate can be obtained, which again can be properly and efficiently processed by dry granulation or direct compression, and which again finally provides single unit dosage forms such as tablets having enhanced dissolution profiles. To this end, the prepared whole dry formulation or granulation of eprosartan mesylate preferably has a water activity of less than 0.62, preferably less than 0.60 and more preferably less than 0.50, respectively determined at room temperature. This can be most effectively accomplished by selecting excipients or additives each satisfying the aforementioned conditions of water activity. When one or more excipients or additives are present whose water activity is higher than the aforementioned range, it is preferred that water activity of other excipients or additives are so low that the final water activity of the prepared whole dry formulation or granulation satisfies the aforementioned conditions of water activity. Paying attention to water activity further contributes to stability of eprosartan mesylate during non-wet processing and long-tem storage. For example it can be ensured that an initially used single form of being either anhydrous or di-hydrated form of eprosartan mesylate can be maintained during processing and storage. Also, variation of dissolution profiles between different production samples, batches or lots can be significantly reduced thereby.

The eprosartan mesylate introduced into the dry formulation or granulation and finally in the single dosage forms and especially in the tablets according to the present invention does maintain a chemically and physically stable form of eprosartan mesylate, especially when in the preferred anhydrous form. As a preparation of a compressed eprosartan mesylate tablet by direct compression or by granulation in a suitable unit dosage form is made effectively feasible according to the present invention, the active principle does not substantially interconvert from anhydrous to hydrated (mono- and di-hydrated) forms. This further enhances stability of the active principle significantly.

A further advantage of the present invention is that solid dosage forms such as tablets can be prepared which comprise only one polymorphic form of the active principle ingredient, eprosartan mesylate. Specifically, when preparing of a solid dosage form of eprosartan mesylate by a dry granulation or direct compression process, preferably using suitable excipients or additives selected from those described above, it is possible to provide eprosartan mesylate in only one form of either anhydrous or dihydrate form. Also in this aspect of the present invention, dry granulation or direct compression is preferably performed with the special conditions described above. As opposed to a wet granulation process, only one form of eprosartan mesylate, namely either anhydrous or dihydrate form, was detected by XRD analysis in the tablets prepared according to the present invention. A significant advantage associated with this feature of the present invention is that the dissolution rate shows less variability. This is particularly beneficial if the anhydrous form of eprosartan mesylate is prepared and not converted to other hydrated forms.

Thus, according to the present invention, interconversion from one form of either anhydrousor di-hydrated forms of eprosartan mesylate to another form can be continuously avoided, not only during processing but throughout long storage times. This is particularly feasible when the single dosage forms, such as tablets, are packed or saved immediately after production within packages and especially blister packs, bottles or press-through package (PTP) that are essentially or totally impermeable towards water vapor and moisture. More preferably, the whole production up to final packaging is performed under conditions of moisture vapor being at most 60% RH (relative humidity), more preferably at most 50 % RH.

Suitable packages are essentially or totally water vapor/moisture impermeable include, but are not limited to foil/foil packs such as aluminum/aluminum blister, HDPE (high density polyethylene bottles), sheets made of plastics having water vapor barrier properties improved such as coated poly(vinyl chloride) or polypropylene, laminated sheets of a polypropylene and a poly(vinylidene fluoride), and blister packs with a - typically thermoformed - blister base part known under the term "tropical blisters". Preferably, the blister packs according to the invention have cold-formed foil/foil blister design and further preferably have black base parts and/or covers, allowing up to 100% protection from moisture, oxygen and light. One element of the foil/foil blister pack comprises a lamination of plastic film (e.g. PVC or PE), adhesive, foil, adhesive, and an outer plastic film. The outer film, which can be PET or PVC, supports the thin aluminum layer and acts as the heat-seal layer. The aluminum layer usually consists of several very thin layers rather than a single thick one. The multiple layers help ensure that pinholes do not go all the way through the foil. They also increase the stretchability of the metal and facilitate the cold-stretching process. These multilayer webs are formed, filled, and sealed on a machine that performs these functions in sequence much as the thermoform-fill-seal machine does except that neither web is heated before the forming step. In the process of making the foil/foil blister pack, during the cold-forming process, the foil is shaped and molded around a plug to form a cavity.

In "tropical blisters", the cover film consists of aluminum or an aluminum/plastics material composite, and a lower sealing tray, which is - typically cold-formed - made from an aluminum/plastics material laminate, is sealed against the rear of the blister base part. Therefore, in a tropical blister, the blister base part with the filling is completely protected by the aluminum films in the cover layer and in the lower sealing tray against the penetration of steam and gases from the external atmosphere.

Moreover, by using eprosartan mesylate present in only one form such as anhydrous form, preferably with controlled particle size, and/or by selecting excipients or additives as described above, it is possible to efficiently and reliably produce single dosage forms like tablets with improved overall characteristics, including acceptable degrees of hardness, friability, weight, shape, disintegration and dissolution. Selecting additives from specific binders, diluents and/or glidants, especially in view of their low water activity specifities and low water or moisture content, can further significantly contribute to an optimal balance of the aforementioned characteristics.

Since the eprosartan mesylate is present in particulate form and in a single form such as only anhydrous form, especially in combination with one or more excipients or additives described above, the obtained dry formulation or granulation exerts sufficient cohesiveness of the formulation ingredients. This facilitates further processing by directly compression or by dry granulation. In particular, sticking and picking problems during compression and granulation processes can be efficiently avoided, enabling a solid dosage form which contains a high dose eprosartan mesylate content ratio, i.e. the eprosartan mesylate itself constitutes a substantial portion of the total weight of the single dosage pharmaceutical formulation like the compressed tablet weight. A substantial variation between content ratios of the active principle in different production batches or production lots can thereby suppressed in a reliable manner. According to the present invention, eprosartan mesylate can beneficially reach an amount of at least 50 wt.-%, preferably at least 60 wt.-%, more preferably at least 65 wt.-%, and particularly at least 70 wt.-% relative to the total amount of the single dosage pharmaceutical formulation. It is a further particular advantage of the present invention that such high drug dose can be reached by dry granulation, thereby avoiding time and expense of wet granulation as well as avoiding a risk of transformation of the stable anhydrous form of the active principle.

The term "dry formulation or granulation" used herein means a preparation wherein the active principle eprosartan mesylate and further ingredients for a pharmaceutical dosage form (especially tablets) have been processed while avoiding typical wet conditions, but using essentially dry or completely dry conditions. This is typically made by using limited amounts of liquids during processing of the active principle, for example to a range clearly below 10 wt.-%, preferably lower than 5 wt.-% and particularly lower than 2 wt.% of the whole formulation mass, or preferably totally avoiding liquids otherwise normally used for agglomeration in wet granulation, such as for example water, ethanol or their combination.

The term "water activity" used herein is defined by the expression a_{w} = p/p₀, where p is the vapor pressure of water in the substance, and p₀ is the vapor pressure of pure water at the same temperature. Water pressure can be measured by suitable water activity meter devices, for example instrument Testo 650 using sensor 0628.0024, both available and sold by EminTech (Helsingborg, Sweden). If not otherwise stated, the water activity referred to in the present specification is determined at room temperature, which is understood herein as the temperature of 22 ± 0.5°C.

The term "set of pharmaceutical formulation samples" used herein means samples of eprosartane mesylate collected from different production samples, batches or lots.

Variation within the set may be calculated from 6 different samples such as 6 tablets. According to the present invention, it is possible to satisfy the variation characteristics even between different production batches/lots.

### Description of further Advantages and Preferred Embodiments of the Invention:

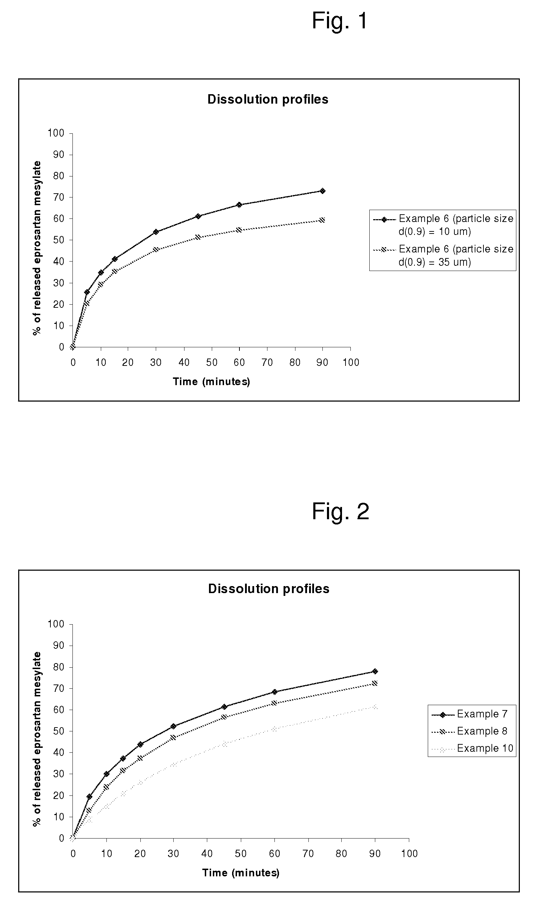

In the following, the present invention will be described in more detail by preferred embodiments and examples while referring to the attached drawings, noting, however, that these embodiments, examples and drawings are presented for illustrative purposes only and shall not limit the invention in any way, wherein
Fig. 1 illustratively shows effects of varying particle sizes of anhydrous eprosartan mesylate on dissolution behavior, exemplified here by relatively slower (example 6-eprosartan mesylate particles with less than 65 v/v % having a particle size of 2 to 27 µm (d (0.9) = 35 µm) and relatively faster (example 6-eprosartan mesylate particles with more than 65 v/v % having a particle size of 2 to 27 µm (d (0.9) = 10 µm) dissolution formulations respectively comprising 600 mg of eprosartan mesylate; and
Fig. 2 illustratively shows effects of the characteristic of low water activity and of the including certain type of additives on dissolution profiles of anhydrous eprosartan mesylate according to example 7 (with mannitol and PEG-4000), example 8 (without manitol) and example 10 (without both mannitol and PEG-4000).

Eprosartan mesylate is hygroscopic (at RH over 60 %). At RH over 60 % eprosartan mesylate anhydrous interconverts to eprosartan mesylate mono- and later to dihydrate, which presents stability problems, and is not inherently compressible. Consequently, there is a need to provide a free-flowing and cohesive eprosartan mesylate composition capable of being directly compressed into high strengths dosage form (with a desirable minimum of 60wt.-% and more preferably as high as at least about 70wt.-% of active principal of the whole dose tablet content), yet with an acceptable and preferably enhanced *in vitro* dissolution profile.

The eprosartan mesylate is provided according to the present invention as a dry formulation or dry granulation. According to preferred embodiments, the eprosartan mesylate used according to the present invention is in anhydrous, particulate and crystalline form. This allows the formulation or granulation to be mixed with appropriately selected excipients or additives, preferably the ones mentioned above, in order to enable direct compression or dry granulation processing of the mixed components. Alternatively, the eprosartan mesylate is in di-hydrated form, and/or is in crystalline form.

The advantages of dry granulation include uniformity of blend, less manufacturing steps involved, elimination of heat and moisture, enabling control of particle size of both the active principle and the agglomerated particles in the course of forming briquets, granules and tablets, and maintaining physical stability. The overall process may involve only weighing of the powders, briquetting or slugging, optionally sieving, milling and blending, and finally compression, hence less cost.

The invention particularly relates to a drug formulation in tablet form containing eprosartan mesylate of specific particle size and in only one form (preferably anhydrous, alternatively mono-hydrate or di-hydrate form) as an active substance and pharmaceutically safe excipients.

The dissolution profile of the drug eprosartan has been evaluated by investigating the influence of variables, and it was found that particle size of active principle and certain properties of additive/excipients, especially in terms of having limited water activity, are relevant factors, and considerations concerning type of additive/excipient, amount of inactive ingredients and physical properties of the tablets can further beneficially influence process and product.

For obtaining suitable particle size to be used for the present invention, appropriate fine powdered, milled, peg-milled or micronized forms of eprosartan mesylate can be used, preferably using anhydrous and crystalline form, to set a particle size that at least 65 % (v/v) fall in a range of 2 to 27 µm as described above. Particle size distribution of eprosartan mesylate can be measured, for example, by laser diffraction (for example using Malvern Mastersizer S).

Eprosartan mesylate has been demonstrated to be relatively stable, however the properties of the substance makes the molecule prone to physical changing when it is in contact with water/moisture. Consequently, to achieve satisfactory physical stability of eprosartan as well as to prevent variability of three different forms (anhydrous, mono- and di-hydrated) and thus variability in dissolution rates in the final solid dosage form, the excipients that are incorporated in the tablet containing eprosartan should be carefully selected in order to have limited water activity as described above. Observing the property of water activity according to the present invention well addresses improved characteristics, as it deals with active water molecules within the pharmaceutical formulation irrespective of its type of binding or association with different excipients or additives. Just the limitation of such active water molecules is a relevant criterion for preventing unwanted interconversion of the three different forms of eprosartan mesylate both during processing and particularly during storage of the final pharmaceutical formulation containing the same. Additionally, the water content in the tablet as a whole is preferably minimized, preferably to less than 5%, more preferably to less than 2 wt. % and particularly to less than 0.5%. Surprisingly, we have observed that using the dry granulation process with appropriate excipient gives the satisfactory physical stability of eprosartan mesylate (just one form such as anhydrate) in the tablets and at the same time dissolution profiles and variability of final product are acceptable. Moreover, performing energy consuming drying in the production of the tablets can be beneficially omitted in the course of simply using direct compression or dry granulation. Especially when using dry granulation, the process of the present invention may include only a low number of steps such as, e.g. briquetting or slugging, optionally, sieving and/or milling, and tabletting, which is in favor of process economy and costs. This together with selecting appropriate excipients and additives as described above makes the whole process of the invention more robust, economical and acceptable.

Thus, it is particularly preferred that the dry formulation or granulation of eprosartan mesylate according to the present invention comprises eprosartan mesylate essentially in anhydrous form only, i.e. without a substantial presence or even with a total absence of monohydrate or dihydrate forms of eprosartan mesylate. The preferred substantially pure anhydrous form of eprosartan mesylate can be identified by X-ray diffraction analysis. Alternatively, another single form of eprosartan mesylate is di-hydrated form, again identifyable by X-ray diffraction analysis.
In order to maintain physical stability over a long period of time, it is preferred that the dry formulation or granulation according to the present invention has a water content of ≤5 wt. %, preferably ≤2 wt. % and particularly ≤0,5 wt. % of the whole dry formulation.

According to the present invention, the amount of filler, binder or other excipients can be limited despite a direct compression or dry granulation process is used while still providing beneficial characteristics of the final tablet. For example, the respective amounts of each excipient or additive may not exceed 10 wt.-%, preferably they may not exceed 7.5 wt.-% or even 6 wt.-%. Further, a majority (more than half of the excipient/additive components) of excipient or additive may not exceed 5 wt.-% each. This in turn means that a high drug load can be achieved with the dry formulation or granulation according to the present invention.

In wet granulation processes, excipient limitation is easier, and it is easier to provide material which has the required flow and cohesive properties to obtain an acceptable solid dosage form which do not tend to segregate.

Surprisingly, the new developed dry formulation/granulation of eprosartan mesylate according to the invention allowed basically the same size of the tablet as that with wet granulation, and at the same time the single dosage pharmaceutical formulation such as the tablets had good physical properties and stability, yet with the possibility of yielding high weight ratios of the eprosartan mesylate. Thus, the present invention meets the need of the prior art to provide an opportunity towards direct compression or dry granulation tablets containing high dose of eprosartan mesylate, thereby avoiding time and expense of wet granulation while maintaining a stable form of the active principle. As explained before, maintaining a single form of being either anhydrous or di-hydrated over a long period of time ensures less variability in dissolution profiles. Providing eprosartan mesylate at a high dose with stable anhydrous eprosartan mesylate is a valuable merit of the present invention, particularly in view of the rather poor flowability characteristics of the drug *per se* being e.g. below 0.15 g/sec and thus the initially associated sticking problem on punches of tablet presses conventionally.

Besides paying attention to selecting particular substances as described above, the excipient or additive besides the active principle or eprosartan mesylate may be suitably selected from binder, diluent, lubricant, dissintegrant, glidant, alone or in combination. Further useful additives may include, alone or in combination, buffer agents, anti-oxidants, colorants, stabilizers, fillers, plasticizers, emulsifiers, preservatives, viscosity-modifying agents, or passifiers, flavouring agents, without being limited thereto.

In order to preferably avoid any wet conditions during dry processing such as briquetting or slugging, sieving and tableting, the excipients or additives mixed with the active principle of eprosartan mesylate are free of agglomeration liquid. Preferably, optionally with the exception of appropriate excipients or additives that do not exist in powdery form, only powdery components should be used for mixing with eprosartan mesylate.

For advantageously improving overall characteristics including flowability, compressibility, uniformity of mass, hardness of tablets, high drug load and drug content uniformity, as well as good dissolution profiles, the following preferably set conditions have been found to be significant:
- The respective amounts of each excipient or additive preferably do not exceed 10 wt.-% relative to the total amount of the final single dosage pharmaceutical formulation.
- The excipient or additive and thus the resulting dry formulation or granulation after mixture with eprosartan mesylate has a low water content, preferably ≤5 wt.-%, or preferably ≤2 wt.-% and particularly ≤0.5 wt.-% of the whole dry formulation or granulation and finally the resulting single dosage pharmaceutical formulation. This can be most effectively accomplished by appropriately selecting suitable excipient or additive substances, or subjecting excipient or additive substances to a water content reduction process such as heating, drying, lyophilisation, water-desorption, or the like.
- The dry formulation or granulation of eprosartan mesylate is defined by a granulation having a particle size distribution in which 40 wt.-% or less of the particles are smaller than 45 µm, preferably 35 wt.-% or less of the particles and more preferably 20 wt.-% or less of the particles are smaller than 45 µm. This can be most effectively accomplished by appropriately controlling pressure force when briquetting is performed, optionally milling or sieving through appropriate mesh size of e.g. 2.0mm (first sieve) and 1.2mm (second sieve), such that the produced granules show such a particle size distribution. Sieve analysis is utilized to measure granulation particle size distribution.
- The dry formulation or granulation providing eprosartan mesylate in only one primary form of being either anhydrous or dihydrate.

In dry granulation methods, the powder particles are aggregated under high pressure. The components of the formulation are compressed at dry status. If the sufficient bonding strength can not be achieved by compression alone, a binder is preferably added, also in a dry state. The initial compression stage can be carried out by specially designed method steps. The first uses a conventional tablet press, a process often referred to as slugging or briquetting. Since the components of the formulation generally will not have the necessary attributes for producing tablets with acceptable characteristics (hardness, friability, weight, shape, disintegration, dissolution), the tablets produced at this stage (the slugs or briquettes) will normally not be of acceptable quality, especially as regards to appearance and weight uniformity. The briquettes are then broken down to form a granular product, which after sieving can then be compressed again to give satisfactory tablets. It was observed that the ease of compressibility of the formulation at the second compression was inversely proportional to the pressure used at the briquetting stage, implying that briquetting at high pressure should preferably be avoided. Examples for suitable conditions at a briquetting stage is that pre-force is maximally 5 kN, main force is maximally 35 kN, which normally results in hardness of briquettes in the range of 70 - 90 N, for example.

Particularly suitable additives to be used according to the present invention are preferably selected from the group consisting of polyethyleneglycol (PEG; Macrogol), preferably PEGs having molecular weights in the range of 400 to 20000; polyvinyl pyrrolidone (PVP) or its cross-linked form crospovidone; silicon dioxide, preferably fumed silica or colloidal silicon dioxide; sugar esters and polyhydroxy-sugars, preferably mannitol; lactose, isomalt, lactitol and dextrose; buffer salts, preferably anhydrous mono-, di- or tri-basic phosphate, more preferably calcium phosphate, calcium sulfate and calcium silicate; hydrophilic, anionic, cationic, zwitterionic and non-ionic surfactants and lipohilic additives such as mono-, di- or triglycerides, polyethoxylated fatty acids, fatty acid esters and oils, preferably sodium oleate, sodium lauryl sulfate and magnesium stearate and polyglycolized glycerides; talc; polysaccharides, preferably starch and celluloses, more preferably hydroxyethylcellulose (HEC), hydroxypropylcellulose (HPC), hydroxypropyl methylcellulose (HPMC), HPMC phthalate, cyclodextrins and carbomers, in particular hydroxypropyl methylcellulose, microcrystalline cellulose (MCC), powdered cellulose; polypeptides such as gelatine; and mixtures thereof. The single dosage pharmaceutical formulation of the present invention may further comprise a cover film or shell, which according to a preferred embodiment is non-hygroscopic as well. In further preferred embodiments, bi- or multi-functional excipients can be used, for example, colloidal silicon dioxide (CSD) admixed with micro-crystalline cellulose (MCC), for example Prosolv^{™}.

In terms of producing tablets with excellent overall characteristics including hardness, friability, weight, shape, disintegration and dissolution, while containing and maintaining anhydrous form of eprosartan mesylate, optimal results have been achieved when selecting as excipient or additive at least one type of PEG having a molecular weight in the range of 400 to 20000 (in particular MACROGOL 4000); isomalt; and mannitol.

The single dosage pharmaceutical formulation exerts beneficial release properties, in particular when releasing eprosartan at a rate of at least 50 % within 30 minutes, more preferably at a more differential rate of at least 60 % within 60 minutes and at least 70 % within 90 minutes, respectively, relative to the original amount of eprosartan in the formulation. The dissolution property is tested using USP apparatus 2, placing the formulations in 1000 ml 0.1 M hydrochloric acid at 37 ± 0.5°C with paddle speed of 50 rpm. Samples are taken from the dissolution vessel at regular time intervals and the concentrations of eprosartan mesylate are analyzed by HPLC. Furthermore, the pharmaceutical formulations show limited variability of a dissolution profile, the said being of below 30 %, preferably below 20% and more preferably below 10 % RSD (relative standard deviation), tested at any time using the aforementioned procedure during dissolution. The RSD is calculated from 6 measurements of different samples or production batches or lots. Moreover, an appropriate hardness of about 80 to 280 N, particularly 100 to 250 N, more particularly 150 to 200 N can be advantageously achieved for the single dosage pharmaceutical formulation according to the present invention. Moreover, for the benefit of high drug load while maintaining an appropriate size of tablets, it is beneficially possible to provide a dry formulation or granulation of eprosartan mesylate having a compressibility of at least 40 %, preferably at least 35 % and in particular at least 30 %. According to the present invention, a directly compressed or dry granulated single dosage pharmaceutical formulation such as tablet is obtained, which has a high dose of drug of at least 60 wt.-% and approved physically and chemically stable form (at ambient temperature and humidity) by way of a more robust, economical and reliable direct compression or dry granulation process, and therefore the single dosage pharmaceutical formulation according to the present invention is particularly useful for the preparation of a medicament, especially for prophylaxis and/or therapy of hypertension, congestive heart failure and renal failure. If desired, the eprosartan mesylate can be combined, within the same single dosage formulation or in combined, yet separate administration forms, with another active principle.

Another advantage of the present invention is that solid dosage forms such as tablets can be prepared which comprise only one polymorphic form of active principle ingredient. Specifically, when preparing of a solid dosage form of eprosartan mesylate by a direct compression, and especially when preparing by dry granulation process, it is possible to maintain eprosartan mesylate in only one of said primary form of being either anhydrous, or dihydrate form. In particular, it has been found to be particularly effective to start with providing either one of anhydrous or dihydrate form of eprosartan mesylate, and to further process this active form with suitable excipients or additives, preferably those described above, to make a solid dosage form such as tablets by direct compression and preferably by dry granulation, in particular with the special conditions described above. The use of a monohydrated form of eprosartan mesylate in dry formulation or granulation is less suitable, as it normally does not satisfy the requirements for formulation having and maintaining only one polymorphic form, as monohydrate form tends to be instable and to interconvert to dihydrate. In addition, in solid dosage form, which had been prepared by wet granulation, both anhydrous and hydrated forms of eprosartan mesylate were detected by XRD analysis. Additionaly, in case of wet granulation the ratio between the anhydrous and the hydrated forms depends on many factors such as amount of moisture, working environment, drying time of granulate and so on. When formulations were obtained by wet granulation in the conventional manner, the resulting mixture contained at least two forms of eprosartan mesylate, namely anhydrous and dihydrate, as the dihydrate arises in situ during wet granulation. That kind of variation may well have influence on *in vitro* and *in vivo* dissolution rate, in particular a high variability of dissolution rates, as well as during tableting process on variability of tablet weight and hardness. On the other hand, only one form of eprosartan mesylate, specifically the anhydrous form, was detected by XRD analysis in the tablets prepared according to the present invention. As a result, the variability of dissolution rates was then remarkably reduced, owing to the presence of only one primary form of eprosartan mesylate.

The following examples are merely illustrative of the present invention and they should not be considered as limiting the scope of the invention in any way, as these examples and other equivalents thereof will become apparent to those versed in the art in the light of the present disclosure, and the accompanying claims.

### Examples

### Examples 1, 2 and 3.

**Table 1. Composition of Eprosartan 600 mg tablets.**

| Constituent | **Examples** | | | Function |
|---|---|---|---|---|
| | **1** | **2** | **3** | |
| Eprosartan mesylate | 735.80 mg | 735.80 mg | 735.80 mg | Active |
| Cellulose microcrystalline (Avicel PH 113) | 86.70 mg | 86.70 mg | 86.70 mg | Binder |
| Ludipress^{™} | 150.00 mg | 150.00 mg | 150.00 mg | Filler and disintegrant |
| Magnesium stearat | 2.50 mg | 10.00 mg | 10.00 mg | Lubricant |
| Macrogol 4000 (PEG-4000) | / | 25.00 mg | / | Binder |
| Glyceryl behenate (Compritol^{™}) | / | / | 25.00 mg | Binder |

### Preparation of Eprosartan tablets

Based on the use of micronized active principle, firstly direct compression as the manufacturing process was investigated. Mixture of active substance and all other excipients, except magnesium stearate, was homogenized and sieved. Then, magnesium stearate was added and homogeneously mixed and tried to compress into tablets, with respective masses of 975 mg for example 1 and 1007.50 mg for examples 2 and 3. According to very low flowability of eprosartan mesylate (below 0.15 g/sec; with sticking on punches) and high percentage of active principle (≥ 73%) in comparison to excipients, direct compression with most commonly used binders in different combinations was not possible.

Further investigation was done by dry granulation process.

The particle size of eprosartan mesylate was that at least 65 v/v% of the particles fell in a range from 2 µm to 27 µm for use in the present invention according to chosen technological procedure and dissolution profiles. This characteristic of the eprosartan mesylate, in particular if it is in a single form only and more particularly if it is in an anhydrous form only, contributes to consistent particle size of the final blend, avoids problems of picking and sticking during later processing, and eliminates problems of content uniformity. A higher particle size slows the release of eprosartan mesylate from the tablets, and also the tableting process is quite problematic according to low compressibility.

Particle size distribution of eprosartan mesylate was measured by laser diffraction using Malvern Mastersizer S. Sample cell was small volume sample dispersion cell MS1, presentation was 3NHE, solvent was hexane, stirrer speed was 2000 rpm. The following procedure was used: Fill the sample cell with hexane, add 100 mg of sodium bis (2-ethylhexyl)sulfosuccinate, align the sizer and measure the background. Add sample into the sample cell until proper obscuration is achieved (10-30%). Analyze when the signal from detectors is stable.

Ingredients selected for further investigations of eprosartan mesylate tablets are shown in Table 2.

**Table 2. Function and approximate amount of chosen excipients.**

| Excipient | Approximate amount (%) | Function |
|---|---|---|
| Lactose monohydrate 70-100 mesh | Up to 4.30 | Diluent |
| Lactose DCL 14 | Up to 8.12 | Diluent |
| Lactose DCL 21 | Up to 3.50 | Diluent |
| Starch 1500 | Up to 7.50 | Disintegrant |
| Crospovidone (Polyplasdon XL) | 2.0 - 4.0 | Disintegrant |
| Cellulose microcrystalline (Avicel PH 113) | Up to 9.42 | Binder |
| Cellulose microcrystalline (Avicel PH 112) | Up to 7.75 | Binder |
| Macrogol 4000 (PEG-4000) | Up to 3.10 | Binder |
| Mannitol | Up to 2.05 | Diluent and binder |
| Mannitol (Pearlitol SD 200) | Up to 1.23 | Diluent and binder |
| Colloidal silicon dioxide (Aerosil 200) | Up to 1.03 | Glidant |
| Magnesium stearate | 0.25 - 2.00 | Lubricant |
| Talc | Up to 2.82 | Glidant |
| Isomalt (GalenIQ721^{™}) | Up to 8.20 | Binder and diluent |

The combination of macrogol-4000, mannitol, cellulose microcrystalline and isomalt with other excipients provided significantly improved compressibility, compared to mixtures for directly compression. On the other hand, mixture of different glidant and lubricant such as colloidal silicon dioxide, magnesium stearate and talc, allowed better flowabillity. Also lactose show radical change in flowabillity of powder during briquetting process according to different particle size as well as different type of cellulose microcrystalline and mannitol. The lactose types are appropriately selected in view of characteristics with respect to particle size, flowability, compressibility, and solubility. A spherical nature and particle size of the obtained granulation, considered alone and preferably in combination, assist in the compressibility of the tablet mix. This results in a mix with greatly reduced inter-particle friction, leading to efficient blending and exceptional flow. Good flowability reduces the variation in the tablet weight and hardness.

Macrogol 4000 and mannitol have a particular influence on compressibility of tablets (35-40%). Furthermore, dissolution profiles are much improved with these two excipients. Still further, addition of glidants and lubricants, in particular with isomalt (such as GalenIQ721^{™}) during briquetting process (internal phase) strongly contributes for acceptable flowability of powder with up to around 81 % of active principle.

During the dry granulation processes using different excipients from the list above, physical stability of the eprosartan mesylate anhydrate was investigated. Eprosartan mesylate in anhydrous form is physically and chemically stable at ambient temperature and humidity, which were used in our laboratories throughout the dry granulation method. The final tablet form and placebo powder were analyzed by X-ray diffraction (XRD) pattern to confirm presence of anhydrous form of eprosartan mesylate.

### Examples 4 and 5.

**Table 3. Composition of Eprosartan 600 mg tablets.**

| Constituent **Internals** | **Examples** | | Function |
|---|---|---|---|
| | **4** | **5** | |
| Eprosartan mesylate | 735.80 mg | 735.80 mg | Active |
| Cellulose microcrystalline (Avicel PH 113) | 94.20 mg | / | Binder |
| Cellulose microcrystalline (Avicel PH 112) | / | 50.00 mg | Binder |
| Lactose monohydrate 70-100 mesh | 40.00 mg | / | Diluent |
| Lactose DCL 14 | / | 81.20 mg | Diluent |
| Starch 1500 | / | 75.00 mg | Disintegrant |
| Crospovidone (Polyplasdon XL) | 20.00 mg | 10.00 mg | Disintegrant |
| Magnesium stearat | 5.00 mg | 3.33 mg | Lubricant |
| Macrogol 4000 (PEG-4000) | 30.00 mg | / | Binder |
| Mannitol | 20.00 mg | / | Diluent and binder |

| Constituent **Externals** | | | |
|---|---|---|---|
| Crospovidone (Polyplasdon XL) | 20.00 mg | 10.00 mg | Disintegrant |
| Colloidal silicon dioxide (Aerosil 200) | / | 3.00 mg | Glidant |
| Magnesium stearat | 10.00 mg | 6.67 mg | Lubricant |
| **Total weight** | 975.00 mg | 975.00 mg | |

Flowability of powder and granulate, compressibility, variability of weight and hardness of the tablets were the main problems during briquetting and tableting according to chosen excipients for examples 4 and 5.

The ingredients were weighted and blended in laboratory blender purpose for 5 min. Subsequently, mixture of powders (internals) was used for compressing into briquettes using a rotary tablet press in a controlled environment. After that, briquettes were grinding twice through 2.5 mm and then 1.2 mm sieve. Afterward, external excipients were screened through a 0.3 mm screen, added to granulate, and mixed for 5 minutes. The resulting final blend was compressed into tablets using a rotary tablet press in a controlled environment. Tablets were compressed at a compression weight of 975 mg using capsule-shaped, biconvex punches.

The manufacturing method of Examples 4 and 5 was repeated in further investigations.

### Example 6.

**Table 4. Composition of Eprosartan 600 mg tablets.**

| Constituent **Internals** | **Examples 6** | Function |
|---|---|---|
| Eprosartan mesylate | 735.80 mg | Active |
| Cellulose microcrystalline (Avicel PH 112) | 79.20 mg | Binder |
| Lactose DCL 14 | 35.00 mg | Diluent |
| Crospovidone (Polyplasdon XL) | 20.00 mg | Disintegrant |
| Magnesium stearat | 5.00 mg | Lubricant |
| Macrogol 4000 (PEG-4000) | 30.00 mg | Binder |
| Mannitol | 15.00 mg | Diluent and binder |
| Talc | 10.00 mg | Glidant |
| Constituent | | |

| **Externals** | | |
|---|---|---|
| Crospovidone (Polyplasdon XL) | 20.00 mg | Disintegrant |
| Colloidal silicon dioxide (Aerosil 200) | 5.00 mg | Glidant |
| Magnesium stearat | 10.00 mg | Lubricant |
| Talc | 10.00 mg | Glidant |
| **Total weight** | 975.00 mg | |

The particle size range of eprosartan mesylate used in example 6 was that at least 65 v/v % of the particles had a size of 2 to 27 µm. Its d(0.9) was ≤ 10 microns. The same combination (type and amount) of excipients, with different particle size such that less than 65 v/v % of the particles had a size of 2 to 27 µm (d(0.9) ≤ 35) were used for a reference analysis of tablets. The higher particle size of the reference sample slowed down the release of eprosartan mesylate from the tablets, and also the tableting process was quite problematic according to low compressibility. Variability of weight and hardness is significantly higher with larger particles of the reference sample.

### Examples 7, 8, 9 and 10.

**Table 5. Composition of Eprosartan 600 mg tablets.**

| Constituent **Internals** | **Examples** | | | | Function |
|---|---|---|---|---|---|
| | **7** | **8** | **9** | **10** | |
| Eprosartan mesylate | 735.80 mg | 735.80 mg | 735.80 mg | 735.80 mg | Active |
| Cellulose microcrystalline (Avicel PH 112) | 50.20 mg | 50.20 mg | 50.20 mg | 50.20 mg | Binder |
| Lactose DCL 14 | 32.00 mg | 44.00 mg | 59.00 mg | 74.00 mg | Diluent |
| Crospovidone (Polyplasdon XL) | 20.00 mg | 20.00 mg | 20.00 mg | 20.00 mg | Disintegrant |
| Magnesium stearat | 6.00 mg | 6.00 mg | 6.00 mg | 6.00 mg | Lubricant |
| Macrogol 4000 (PEG-4000) | 30.00 mg | 30.00 mg | 15.00 mg | / | Binder |
| Mannitol (Pearlitol SD 200) | 12.00 mg | / | / | / | Diluent and binder |
| Talc | 12.50 mg | 12.50 mg | 12.50 mg | 12.50 mg | Glidant |

| Constituent **Externals** | | | | | |
|---|---|---|---|---|---|
| Crospovidone (Polyplasdon XL) | 15.00 mg | 15.00 mg | 15.00 mg | 15.00 mg | Disintegrant |
| Cellulose microcrystalline (Avicel PH 112) | 27.50 mg | 27.50 mg | 27.50 mg | 27.50 mg | Binder |
| Colloidal silicon dioxide (Aerosil 200) | 5.00 mg | 5.00 mg | 5.00 mg | 5.00 mg | Glidant |
| Magnesium stearat | 14.00 mg | 14.00 mg | 14.00 mg | 14.00 mg | Lubricant |
| Talc | 15.00 mg | 15.00 mg | 15.00 mg | 15.00 mg | Glidant |
| **Total weight** | 975.00 mg | 975.00 mg | 975.00 mg | 975.00 mg | |

Physical properties of powders and granulates over and above dissolution profiles for mixtures of Examples 7, 8, 9 and 10 have shown that macrogol 4000 and mannitol have very significant influence (*p < 0.05*; Student's *t* test) on briquetting and tableting processes, i.e. relating technically to process characteristics, as well as *in vitro* studies, especially dissolution profile.

### Example 11.

**Table 6. Composition of Eprosartan 600 mg tablets.**

| Constituent **Internals** | **Examples 11** | Function |
|---|---|---|
| Eprosartan mesylate | 735.80 mg | Active |
| Lactose DCL 21 | 34.00 mg | Diluent |
| Isomalt (GalenIQ721) | 50.20 mg | Binder and diluent |
| Crospovidone (Polyplasdon XL) | 20.00 mg | Disintegrant |
| Magnesium stearat | 5.00 mg | Lubricant |
| Macrogol 4000 (PEG-4000) | 30.00 mg | Binder |
| Mannitol (Pearlitol SD 200) | 10.00 mg | Diluent and binder |
| Talc | 12.50 mg | Glidant |
| Colloidal silicon dioxide (Aerosil 200) | 5.00 mg | Glidant |

| Constituent **Externals** | | |
|---|---|---|
| Crospovidone (Polyplasdon XL) | 15.00 mg | Disintegrant |
| Colloidal silicon dioxide (Aerosil 200) | 5.00 mg | Glidant |
| Cellulose microcrystalline (Avicel PH 112) | 27.50 mg | Binder |
| Magnesium stearat | 10.00 mg | Lubricant |
| Talc | 15.00 mg | Glidant |
| **Total weight** | 975.00 mg | |

### Example 12.

Dissolution properties of eprosartan mesylate obtained in the above described Examples were evaluated by *in vivo* relevant dissolution test. USP apparatus 2 was used for testing. 1000 mL of artificial juice (0.1 M HCl; ) is placed in the dissolution vessel, mixed with a paddle at 50 rpm and kept at 37 ± 0.5°C. Samples were taken from the dissolution vessel at regular time intervals and the concentrations of eprosartan mesylate were analyzed by HPLC.

Figure 1 shows respective dissolution profiles from the slower (Example 6 - eprosartan mesylate with less than 65 v/v % of the particles having a size of 2 to 27 µm ) and faster (Example 6 - eprosartan mesylate with at least 65 v/v % of the particles having a size of 2 to 27 µm ) dissolution formulations comprising 600 mg of eprosartan messylate.

According to dissolution testing results for the same formulation with active substances which differed by particle size, it was confirmed that the particle size has significant influence on dissolution profile. Difference at the end point is higher than 5%. Moreover, flowability of dry granulates which were made by these two different types of active principle was worse with granulate contained larger particle size then with granule with fine micronized eprosartan mesylate powder. Accordingly, compressibility and variability (tablet weight and hardness) were significantly better with smaller particle size.

Figure 2 shows dissolution profiles of eprosartan mesylate for Example 7 (with mannitol and PEG-4000), Example 8 (without mannitol) and Example 10 (without both mannitol and PEG-4000).

Dissolution profiles for mixtures of Examples 7, 8, and 10 have shown that macrogol 4000 (PEG-4000) and mannitol have very significant influence on dissolution profiles (*in vitro* studies). Still further, without both of these excipients physical properties of powder mixture for briquetting and granulate for tableting are significantly worse, according to very low compressibility which brings out weight and hardness variability of tablets.

### Example 13.

Samples of tablets according to the present invention were produced as described in the above Examples, with the difference that eprosarten mesylate dihydrate form was provided and processed as API. Resulting tablets achieved an API load of more than 50 wt.-%, and had the following characteristics:
Flowability (final mixture for tabletting): 0.6 g/s.
Moisture content (final mixture for tabletting) : 5.6%
Compressibility (final mixture for tabletting): approximately 25%.
Hardness (briquettes): 50 - 80 N.
Hardness (tablets): 160 -240 N.

### Example 14.

In this example, the tablets prepared according to the present invention have been investigated and applied in various further ways.

### (1) Only one form of eprosartan mesylate:

Diffractograms were collected on X'Pert PRO MPD diffractometer using CuKα radiation. Data were recorded from 2 do 40 ° 2θ; in steps of 0.033 °2θ; with the integration times 50 s (routine testing) and from 4 do 20 ° 2θ; in steps of 0.033 °2θ; with the integration times 2000 s (to achieve lower LOD). The range for measurements with longer integration times was chosen according to the known diffractograms of eprosartan mesylate crystalline forms, which all have characteristic peaks in the carefully measured range between 4 and 20 ° 2theta.

Only anhydrous form of eprosartan mesylate was detected in the tablets prepared according to the present invention.

### (2) Water content of eprosartan mesylate dry granulations and tablets:

Furthermore, dry granulations and tablet were checked every time during and after the production with respect to water content. The method applied was the loss on drying (15 minutes at 80°C). It was found that water content was always lower than 1.2%. Accordingly, after appropriate packing (sealed pack, black plastic bag or *vitrum nigrum*), water content after 6 months was still below 1.2%. In addition, the water activity measurements always showed the water activity of a formulation of less than 0.62.

### (3) Particle size of eprosartan mesylate dry granulations and hardness of tablets:

A dry granulation according to the present invention was subjected to briquetting applying a pre-force of up to 5 kN, a main force of up to 35 kN.
Subsequently, briquetts have been sieved throughout a first sieve of 2.0 mm and a second sieve of 1.2 mm. Particle size distribution after sieving process is shown in Table 1 below:

**Table 1: Particle size distribution of dry granulation according to the present invention after briquetting and sieving process, before tabletting**

| Sieve (µm) | Percentage (%) |
|---|---|
| 1000 | 2.55 |
| 710 | 11.42 |
| 500 | 17.11 |
| 400 | 7.09 |
| 315 | 7.26 |
| 200 | 10.81 |
| 100 | 25.19 |
| 45 | 16.44 |
| Bottom | 2.12 |
| Σ | 100.00 |

It has been shown that shape and size of particles are not the same before tabletting process. According to our investigations, it is best if the there are less than 40 wt.-% of particles under 45 µm, particularly less than 35 wt.-%, more particularly less than 20 wt.-%. Otherwise, small particles cause sticking and picking during further processing.

The standard method used for testing tablet hardness, as referred to in the present specification, is compression testing, which is normally used for research & development and for quality control. The tablet is placed between two jaws that crush the tablet. The machine measures the force applied to the tablet and detects when it fractures. Although compressive force is applied to the tablet, tablets usually fail in a tensile manner, along the diameter of the tablet at right angles to the applied force. Newton (N) - The Newton is the SI unit of force and is the unit that should be used for tablet hardness testing. 9.807 Newtons = 1 kilogram. When tablet hardness is referred to, it actually means - the compressive strength of the tablet.

Hardness ranges feasible according to the present invention are 90-280 N, particularly 100-250 N, more particularly 150-200 N.

### (4) Dissolution variability:

Tablets of the present invention as well as of a commercial comparison product were subjected to dissolution testing with attention to variability of dissolution. The results with a comparison relative to various tablets produced according to the present invention are shown in Tables 2 and 3.

**Table 2: Variability of dissolution profile**

| | Commercial | Example 6 | Example 6 |
|---|---|---|---|
| Time | Comparison product | (10 µm) | (35 µm) |
| (min) | RSD | RSD | RSD |
| 5 | 32.2 | 6.6 | 15.2 |
| 10 | 10.3 | 6.6 | 18.6 |
| 15 | 7.3 | 5.8 | 20.2 |
| 30 | 4.2 | 5.6 | 20.7 |
| 45 | 3.2 | 6.0 | 21.0 |
| 60 | 2.8 | 5.7 | 20.3 |
| 90 | 1.9 | 5.1 | 19.7 |

| | | | |
|---|---|---|---|
| RSD is relative standard deviation | | | |

**Table 3: Variability of dissolution profile**

| | Commercial | Example 7 | Example 8 | Example 10 |
|---|---|---|---|---|
| | Comparison product | RSD | RSD | RSD |
| Time | | | | |
| (min) | RSD | | | |
| 5 | 32.2 | 8.2 | 27,9 | 24,1 |
| 10 | 10.3 | 3.0 | 16,0 | 15,9 |
| 15 | 7.3 | 1.9 | 8,6 | 19,1 |
| 30 | 4.2 | 1.5 | 4,1 | 15,3 |
| 45 | 3.2 | 1.3 | 2,6 | 14,1 |
| 60 | 2.8 | 0.7 | 1,6 | 12,4 |
| 90 | 1.9 | 0.5 | 0,6 | 10,6 |

It has been confirmed that the pharmaceutical formulations (tablets) according to the present invention have superior dissolution profiles over the comparison product, especially in terms of lower variability among different samples of a pharmaceutical formulation (tablet) collection, which could have beneficial effect in *in vivo* applications. According to the present invention, it is made feasible that the variability of dissolution is below 20 %, preferably below 10 % relative standard deviation (RSD), measured using USP apparatus 2, placing the formulations in 1000 ml 0.1 M hydrochloric acid at 37 ± 0.5°C with paddle speed of 50 rpm after 5, 10 or 15 minutes. It is particularly feasible and favorable that the RSD is below 30 %, preferably below 20% and more preferably below 10% RSD all time during a dissolution test under the aforementioned measurement conditions of using USP apparatus 2, placing the formulations in 1000 ml 0.1 M hydrochloric acid at 37 ± 0.5°C with paddle speed of 50 rpm. The fact the pharmaceutical formulations (tablets) according to the present invention are superior is mainly attributed to the effects which are contributed by the features (i) only one form of active principle (here: anhydrous eprosartan mesylate only); (ii) particle size of the active principle eprosartan mesylate; and (iii) type of certain excipients.

## Claims

1. A dry formulation or granulation of eprosartan mesylate, comprising eprosartan mesylate in particulate form, wherein at least 65 v/v % eprosartan mesylate particles fall in a particle size range of from 2 to 27 µm.

2. A dry formulation or granulation of eprosartan mesylate, comprising eprosartan mesylate combined with an excipient, wherein the excipient at least comprises a PEG having molecular weight in the range of 400 to 20000 and mannitol.

3. The dry formulation or granulation of eprosartan mesylate according to claim 2, wherein the excipient further comprises at least lactose or isomalt, or mixtures thereof.

4. A dry formulation or granulation of eprosartan mesylate, comprising eprosartan mesylate in particulate form mixed with excipients or additives, wherein the dry formulation or granulation of eprosartan mesylate has a water activity of less than 0.62.

5. The dry formulation or granulation of eprosartan mesylate according to any one of the preceding claims, wherein none of excipients or additives added to the formulation or granulation are combined with agglomeration liquid and all the excipients or additives are added in the form of powders optionally with the exception of appropriate excipients or additives that do not exist in powdery form.

6. The dry formulation or granulation of eprosartan mesylate according to any one of the preceding claims, comprising excipient or additive whose respective amount does not exceed 10 wt.-% each.

7. The dry formulation or granulation of eprosartan mesylate according to any one of the preceding claims, which is defined by a granulation having a particle size distribution in which 40 wt.-% or less of the particles are smaller than 45 µm, preferably 35 wt.-% or less of the particles and more preferably 20 wt.-% or less of the particles are smaller than 45 µm.

8. The dry formulation or granulation of eprosartan mesylate according to any one of the preceding claims, wherein said eprosartan mesylate is either in dihydrate form or, preferably, in anhydrous form, more preferably in the absence of another form of eprosartan mesylate.

9. A pharmaceutical formulation, obtained from a dry formulation or granulation of eprosartan mesylate according to any one of the preceding claims by direct compression or dry granulation.

10. A pharmaceutical formulation comprising eprosartan mesylate, which has a dissolution profile by releasing eprosartan at a rate of at least 50 % within 30 minutes, relative to the original amount of eprosartan in the formulation, tested using USP apparatus 2, placing the formulations in 1000 ml 0.1 M hydrochloric acid at 37 ± 0.5°C with paddle speed of 50 rpm.

11. A set of pharmaceutical formulation samples each comprising eprosartan mesylate as an active ingredient, wherein the eprosartan mesylate shows a dissolution profile with a variability of dissolution from the different pharmaceutical formulation samples of the set of below 30 %, preferably below 20% and more preferably below 10% relative standard deviation at all time during dissolution, measured using USP apparatus 2, placing the formulations in 1000 ml 0.1 M hydrochloric acid at 37 ± 0.5°C with paddle speed of 50 rpm.

12. The pharmaceutical formulation according to claim 10, or the set of pharmaceutical formulation samples according to claim 11, each comprising eprosartan mesylate in only either anhydrous or dihydrate form as an active ingredient.

13. A process for the preparation of a pharmaceutical formulation of eprosartan mesylate, **characterized by** providing eprosartan mesylate in only one primary form of being either anhydrous or dihydrate, and subjecting said primary form to a direct compression or dry granulation process while maintaining said only one primary form.

14. The process according to claim 13, **characterized by** providing and subsequently maintaining only anhydrous eprosartan mesylate.

15. A package comprising:
at least one single dosage form comprising eprosartan mesylate in only one form of either anhydrous or dihydrate form, which single dosage form is in a dry granulated or direct compressed dosage form;
wherein said at least one single dosage form is packed in a package sealed against vapor and moisture permeation.
